Europäisches Patentamt

(19) European Patent Office

Office européen des brevets

(11) Publication number: **0 282 318**
**A2**

# (12) EUROPEAN PATENT APPLICATION

(21) Application number: **88302117.2**

(22) Date of filing: **10.03.88**

(51) Int. Cl.⁴: **C 07 K 13/00**
**C 12 P 21/02, C 12 N 15/00,**
**A 61 K 37/36**

(30) Priority: **12.03.87 US 24838    24.02.88 US 153953**

(43) Date of publication of application:
**14.09.88 Bulletin 88/37**

(84) Designated Contracting States:
**AT BE CH DE ES FR GB GR IT LI LU NL SE**

(71) Applicant: **Amgen Inc.**
**1900 Oak Terrace Lane**
**Thousand Oaks, California 91320 (US)**

(72) Inventor: **Souza, Lawrence M.**
**1302 Witherspoon Drive**
**Thousand Oaks California 91360 (US)**

**Rudman, Christopher G.**
**833 Corbett Avenue**
**San Fransisco California 94131 (US)**

(74) Representative: **Brown, John David et al**
**FORRESTER & BOEHMERT Widenmayerstrasse 4/I**
**D-8000 München 22 (DE)**

(54) **Bovine growth hormone analogs.**

(57) An analog of growth hormone, specifically the analog having a methionine residue at its N terminus and including residues identical to the residues at positions 1 through 32 and 40 through 191 in the amino acid sequence of bovine growth hormone (i.e., $rbGH_{1-32,40-191}$), retains the diabetogenic, insulin-sparing and lipolytic properties of bovine growth hormone while being capable of improving growth in mammals and in salmon while also being capable of a marginal increase in milk production in mammals.

**Description**

BOVINE GROWTH HORMONE ANALOGS

Background

The present invention relates to a class of analogs of bovine growth hormone. In particular, the present invention relates to a class of recombinantly-produced analogs of bovine growth hormone, wherein one or more residues at positions 33 through 39 in the amino acid sequence of naturally occurring bovine growth hormone are deleted. The invention further relates to compositions containing such analogs and to the use of such compounds and compositions.

The pituitary gland of normal mammals produces and secretes into the bloodstream a substance called growth hormone ("GH"). The amino acid sequences of human ("hGH"), bovine ("bGH"), and porcine ("pGH") growth hormones are similar. See Dayhoff, Atlas of Protein Sequence and Structure, Volume 5, Supplement 6, National Biomedical Research Foundation, Washington, 120-121 (1976); and Seeburg et al., DNA, 2, 37-45 (1983). The amino acid and nucleotide sequences of salmon growth hormone ("sGH") is also known, Sekine et al., Proc. Nat'l. Acad. Sci. (USA), 82, 4306-4310 (1985). Based upon an alignment of the sequences of bGH, hGH, pGH, and sGH which provides the highest degree of homology among these growth hormones, certain highly conserved regions may be identified. See e.g., Dayhoff, supra, and Sekine et al., supra.

At least in vivo, growth hormone promotes construction of protein from amino acids, an initial fall in plasma glucose upon administration, a gradual rise in plasma glucose after the initial fall, and a breakdown of fats into fatty acids. These actions associated with growth hormone are respectively referred to as growth promotion (ie., weight gain), insulin-sparing, diabetogenic and lipolytic effects. An antilipolytic effect has also been reported, but this appears to be a facet of the insulin-like activity of the hormone. Goodman, Metabolism, 19, 849-855 (1970).

In addition, growth hormones are similar in structure to lactogenic hormones and are capable of inducing similar effects. For example, human growth hormone differs from the human placental lactogen at about 15% of its residues. Wallis et al., in Growth Hormone and Related Peptides, Pecile et al., eds., Excerpta Medica, Amsterdam, 1-13 (1976). Human growth hormone differs from human prolactin at about 25% of its residues. Wallis et al., supra. Subcutaneous injection of bGH or recombinant bGH ("rbGH") increases milk yield in cows, goats and sheep. Eppaird et al., J.Dairy Sci. 68, 1109-1115 (1985); Bauman et al., J. Dairy Sci., 68, 1352-1362 (1985); Hart, Proc. Nutr. Soc., 42, 181-194 (1983); and see Hart et al., Biochem. J., 218, 573-581 (1984).

The isolation of growth hormone from pituitaries involves lysing pituitary cells associated with production of the hormone. However, the lysing of cells releases proteolytic enzymes (proteases) which may cleave at least some of a naturally occurring pituitary growth hormone into fragments. Furthermore, once secreted into the bloodstream, naturally-occurring pituitary growth hormone is exposed to proteases which may cleave the naturally occurring pituitary growth hormone into the same or into different fragments. A major area of investigation for growth hormone fragment research is directed at a determination of whether naturally occurring growth hormone or its fragments or both give rise to the actions associated with growth hormones which have been extracted or which are circulating in the bloodstream. In this regard, it may be noted analogs of human growth hormone rendered resistant to digestion by the protease trypsin by chemically modifying lysine or arginine residues possess significant, albeit attenuated, growth-promoting, diabetogenic and insulin-like activities. Cameron et al., Biochim. Biophys. Acta, 254-260 (1985). Nevertheless, discrete portions ("domains") of the naturally occurring growth hormone molecule are believed to be responsible for one or another of the effects of the growth hormone. To the extent that responsibility for the actions of naturally occurring growth hormone may be localized in this way, fragments and analogs may be produced in which the protein-synthetic, insulin-sparing, diabetogenic and lipolytic effects are selectively altered.

As used hereinafter, the positions of amino acid residues present in fragments or analogs of bovine growth hormone are identified in a subscript wherein numbers indicate the presence of the residues found at the same positions in the corresponding naturally occurring bovine growth hormone and wherein deletions are indicated by a comma. For example, naturally occurring bovine growth hormone is represented by $bGH_{1-191}$.

A 20,000-dalton variant ("20K") of hGH (22,000-dalton) which may be isolated from pituitaries and which corresponds to $hGH_{1-31,47-191}$, promotes growth in hypophysectomized rats, is not hyperglycemic or hyperinsulinemic in dogs, is neither insulin-sparing nor lipolytic in vivo or in vitro, and is less reactive in radioimmunoassays for hGH than is hGH itself. Lewis et al., J. Biol. Chem., 253, 2679-2687 (1978); Frigeri et al., Biochem. Biophys. Res. Commun., 91, 778-782 (1979); Lewis et al., Biochem. Biophys. Res. Commun., 92, 511-516 (1980); and Lewis et al., Endocr. Res. Commun., 8, 155-164 (1981). This 20K variant of hGH is a product of post-transcriptional modification. Lewis et al., Biochem. Biophys. Res. Commun., supra. It may be the case that the 20K variant may be a more important growth promoter than would be predicted from its in vitro bioactivity due to its tendency to dimerize and thus escape renal degradation. Baumann et al., Endocrinology, 117, 1309-1313 (1985).

Fragments of hGH which include residues deleted from 20K hGH have been prepared. Although none of these fragments are reported to promote growth, some exhibit properties of potential relevance to the diabetogenic and lipolytic properties of growth hormone.

A synthetic fragment corresponding to residues 31-44 of hGH is lipolytic in vivo in starved animals and in vitro [Yudaev, et al., Biokhimiya, 41, 843-846 (1976)] but stimulates glucose uptake (i.e. was insulin-sparing) only after in vitro preincubation in the absence of GH, a non-physiological state. Yudaev, et al., Biochem. Biophys. Res. Commun., 110, 866-872 (1983). Some peptides analogs of hGH are diabetogenic but an analog of hGH$_{52-77}$ is not. Lostroh, et al., Diabetes, 27, 597-598 (1978). A peptide consisting of hGH$_{20-41}$ is devoid of activity. Reagan, Diabetes, 27, 883-888 (1978). A peptide consisting of hGH$_{1-36}$ is devoid of effect on blood glucose or on growth. Chillemi, et al., in Growth Hormone and Related Peptides, Pecile, et al., eds., Excerpta Medica, Amsterdam, 50-63, (1976).

However, a peptide corresponding to hGH$_{32-46}$ causes a decrease in serum free fatty acids, and is insulin-sparing when coadministered with insulin in vitro [Frigeri et al., in Proceedings, 64th Annual Meeting of the Endocrine Society, San Francisco, 101 (Abstract 88) (1982)] and in vivo [Rudman, U.S. Patent No. 4,558,033, and Stevenson et al., Diabetes, 33, 149A (Abstract No. 572) (1984)]. Fragments and analogs (involving substitution of heterologous amino acids or stereoisomers) of hGH$_{32-46}$ are also insulin-sparing when coadministered with insulin in vivo. Jones et al., copending and coassigned U.S. Patent Application Serial No. 501,024.

## SUMMARY OF THE INVENTION

The present invention relates to a class of recombinantly derived bovine growth hormone analogs which retains the biological activity and properties of naturally occurring bovine growth hormone while increasing the growth rate, feed efficiency, lypolysis and/or milk yields.

In particular, the present invention relates to a recombinant bovine growth hormone analog represented by the amino acid sequence:

Z-bGH$_{1-32}$-(X)$_n$-bGH $_{40-191}$ (I)

wherein

n is 0 or 1
Z is hydrogen or methionine; and
X is a peptide of an amino acid residue comprising

-GLU-ARG-THR-TYR-ILE-PRO-GLU-
wherein one or more of the amino acids are deleted; and allelic versions thereof.

The present invention further relates to processes of construction of various replicable cloning vehicles harboring the DNA sequences as well as expression vehicles harboring DNA sequences useful to direct the production of the bGH analogs of the present invention in transformed bacterial or transfected cell lines. In addition, the present invention provides for a gene encoding the bGH analogs of the present invention of bGH having the above-described amino acid sequence. The present invention also encompasses the various relicable cloning vehicles, expression vehicles, and transformed bacterial or cell cultures, all harboring the altered genetic information necessary to effect the production of the bGH analogs of the present invention.

The bGH analogs of the present invention are produced in substantially pure form and therefore exist essentially free of other proteins of bovine origin. The bGH analogs may be formulated with other conventional carriers and adjuvants, including other proteins, for example, serum albumin, to yield acceptable compositions so as to facilitate efficacious delivery to a host animal.

The present invention also provides a method for promoting growth in an animal comprising administering to an animal an effective dose of a bovine growth hormone analog of the present invention or composition containing such bovine growth hormone analog.

In addition, the present invention provides a method for promoting milk production in a animal comprising administering to the animal an effective dose of a bovine growth hormone analog of the present invention.

## Brief Description of the Drawings

Fig. 1 is a graphic depiction of weight gain in Coho salmon achieved upon administration with a 21K bGH analog according to the present invention;

Fig. 2 is a graphic depiction of increase in length of Coho salmon achieved upon administration with 21K bGH analog of the present invention;

Fig. 3 is a graphic depiction of the performance of a 21K bGH of the present invention in a radioimmunoassay for bGH;

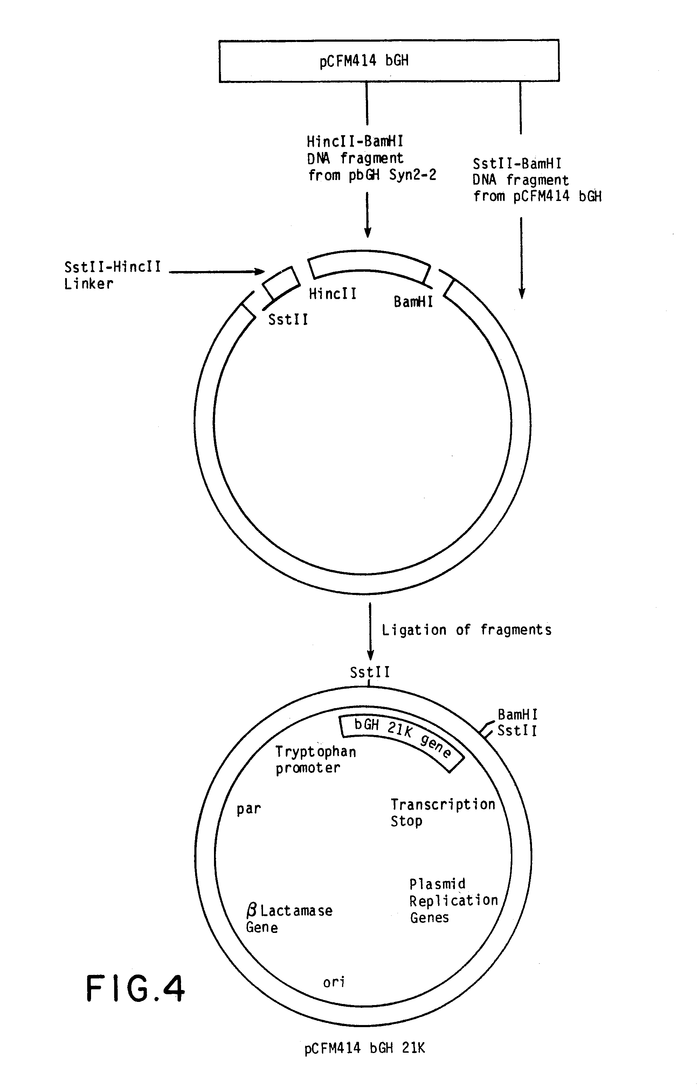

Fig. 4 is a diagram of the construction of pCFM414bGH21K, illustrating the components utilized in plasmid construction, and

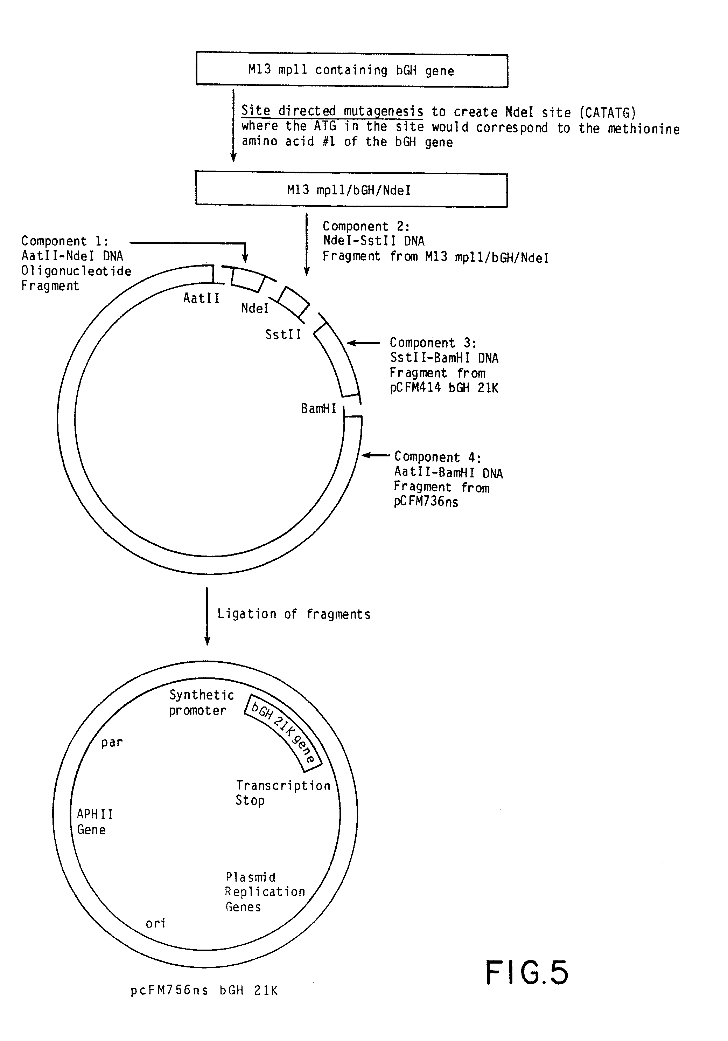

Fig. 5 is a diagram of the construction of pCFM756nsbGH21K. This drawing illustrates the components utilized in plasmid construction.

## Detailed Description

As discussed above, physiological activities of growth hormone may be attributed to the different domains of the intact polypeptide. The activities may also be due to a particular folding or modification of the intact polypeptide, to the release of mediating factors, or to "contamination" by other pituitary peptides, e.g. $\alpha$ - and $\beta$ -lipotropin which themselves can be responsible for lipolytic activity [Kuhn et al., J. Clin. Endocrinol. Metab., 56, 1338-1340 (1983)]. Frigeri et al., Hormone Res., 17, 197-201 (1983).

One way to separate the effects of contaminants from the effects of purified hormones is to examine the activities of a growth hormone which is produced in isolation from other pituitary components, e.g. recombinant bGH ("rbGH"). The gene for bGH has been sequenced and has been expressed in prokaryotic and eukaryotic cells in a variety of forms. Keshet et al., Nucleic Acids Res., 9, 19-30 (1981); Woychik et al., Nucleic Acids Res., 10, 7197-7210 (1982); Seeburg et al., DNA, 2, 37-45 (1983); Kopchick et al., DNA, 4, 23-31 (1985); and George et al., DNA, 4, 273-281 (1985). Recombinant bGH is immunologically identical to nbGH in a radioimmunoassay, has about the same growth-promoting activity in the dwarf mouse bioassay, and possesses somewhat less diabetogenic activity in insulin tolerance tests on sheep. Hart et al., Biochem. J., 224, 93-100 (1984).

The present invention provides purified and isolated polypeptide products having one or more of the biological properties (e.g., immunological properties and in vitro biological activity) and physical properties (e.g., molecular weight) of naturally-occurring bGH including allelic variants thereof. These polypeptides are also characterized by being the product of chemical synthetic procedures or of procaryotic or eucaryotic host expression (e.g., by bacterial, yeast, higher plant, insect and mammalian cells in culture) of exogenous DNA sequences obtained by genomic or cDNA cloning or by gene synthesis. The products of typical yeast (e.g., Saccaromyces cerevisiae) or procaryote [e.g., Escherichia coli (E. coli)] host cells are free of association with any mammalian proteins. The products of microbial expression in vertebrate (e.g., non-human mammalian and avian) cells are free of association with any human proteins. Depending upon the host employed, polypeptides of the invention may be glycosylated with mammalian or other eucaryotic carbohydrates or may be non-glycosylated. Polypeptides of the invention may also include an initial methionine amino acid residue (at position -1).

As used herein the term "peptide of an amino acid residue" refers to an amino acid residue GLU-ARG-THR-TYR-ILE-PRO-GLU wherein one or more amino acids have been deleted. For the purposes of the present invention, the deletion of the amino acids in the peptides thus described may be sequential or random.

As employed herein, the term "manufactured" as applied to a DNA sequence or gene shall designate a product either totally chemically synthesized by assembly of nucleotide bases or derived from the biological replication of a product thus chemically synthesized. As such, the term is exclusive of products "synthesized" by cDNA methods of genomic cloning methodologies which involve starting materials which are initially of biological origin.

As used herein, the term "allelic versions" refers to modifications of one or more amino acids in the sequence of the bGH analogs of the present invention without altering the biological activity of the analog. Such allelic versions are readily ascertained by one of ordinary skill in the art.

The recombinant $bGH_{1-191}$ (that is intact rbGH) proved to be growth promoting in both hypophysectomised rats and in dwarf mice Wallis, et al., J. Endocrinol.. 56, 235-243 (1973). The recombinant $bGH_{1-32,40-191}$ analog was found to be growth promoting in both rodent species. The recombinant $bGH_{1-191}$ was at least as effective as naturally occurring $bGH_{1-191}$ when overall weight gain was maintained.

A preferred bGH analog of the present invention comprises a bGH analog of formula (I) wherein n is 0 ("$bGH_{1-32, 40-191}$"). Additional preferred bGH analogs are represented in TABLE 1:

TABLE 1

| Analog | n | X |
|---|---|---|
| $bGH_{1-35,39-191}$ | 1 | -GLU-ARG-THR-GLU- |
| $bGH_{1-37,39-191}$ | 1 | -GLU-ARG-THR-TYR-ILE-GLU- |
| $bGH_{1-32,35-38,40-191}$ | 1 | -THR-TYR-ILE-PRO- |
| $bGH_{1-33,35-191}$ | 1 | -GLU-THR-TYR-ILE-PRO-GLU- |

The protocol employed to prepare the manufactured gene encoding a recombinant $bGH_{1-191}$ is generally described in the disclosure of Alton, et al., PCT Publication No. WO83/04053, which is incorporated by reference herein. The genes were designed for initial assembly of component oligonucleotides into multiple duplexes which, in turn, were assembled into 2 discrete sections. These sections were designed for ready amplification and, upon removal from the amplification system, could be assembled sequentially or through a multiple fragment ligation in a suitable express vector.

The compositions and methods of the present invention utilize an effective amount or dose of the bovine growth hormone analogs of the present invention. As used herein the term "effective amount or dose" of the bovine growth hormone analog refers to an amount of bovine growth hormone to be administered to an animal in order to produce an increase in growth or related properties, i.e., feed efficiency, leaner carcus composition, increased milk production and the like. Such effective amounts or doses are readily ascertained by one of ordinary skill in the art.

The following examples serve to further illustrate the embodiments of the present invention.

Example 1

This example describes the preparation of a manufactured gene encoding 22K rbGH including E. coli preference condons.

A gene encoding 22K rbGH was constructed from two synthetic DNA duplexes. These duplexes, a 344 bp Xbal - HindIII fragment (Fragment A) and a 254 bp HindIII - Sall fragment (Fragment B) were obtained by enzymatic assembly of 26 and 20 synthetic oligodeoxyribonucleotides respectively and then sequentially cloned into a pBR 322 derived plasmid. Fragment A includes oligonucleotides 19 through 44 represented in Table 2 and Fragment B includes oligonucleotides 1B through 22 represented in Table 2. Table 2 also represents the entire nucleotide sequence of the manufactured gene.

The Xbal to HindIII fragment formed by Section A is ligated into an M13mp11 phage vector opened with Xbal and HindIII. The vector is then reopened by digestion with HindIII and Sall followed by ligation with the HindIII to Sall fragment formed by Section B. At this stage, Sections A and B have been joined in proper orientation. The vector containing Sections A and B is digested with Xbal and Sall. The fragment resulting from this digestion is ligated into a pBR 322 derived plasmid. The product of this reaction is an expression plasmid containing a continuous DNA sequence, as shown in Table 3, encoding the entire recombinant $bGH_{1-191}$ polypeptide with an amino terminal methionine codon (ATG) for E. coli translation initiation.

Xba I end

```
          10          20          30          40          50
CTAGAGAATGGCTTTTC CAGCAATGTC TCTGTCCGGT CTGTTCGCTA ACGCGGTACT
TCTTACCGAAAAG GTCGTTACAG AGACAGGCCA GACAAGCGAT TGCGCCATGA

          60          70          80          90          100
GCGTCCTCAG CATCTGCACC ACTTACCCGC GGACACTTTC AAAGAATTTG
CGCACGGAGTC GTAGACGTGG TCAATCGGCG CCTGTGAAAG TTTCTTAAAC

          110         120         130         140         150
AACGTACCTA CATCCCAGAA GGTCAACGCT ACTCTATCCA GAACACTCAG
TTGCATGGAT GTAGGGTCTT CCAGTTGCGA TGAGATAGGT CTTGTCAGTC

          160         170         180         190         200
GTTGCTTTCT GCTTTTCTGA GACTATTCCG GCACCAACCG GTAAAAACGA
CAACGAAAGA CGAAAAGACT CTGATAAGGC CGTGGTTGGC CATTTTTGCT

          210         220         230         240         250
GGCACAGCAG AAATCCGATC TGGAGCTCCT GCGTATCTCT CTGTTACTGA
CCGTGTCGTC TTTAGGCTAG ACCTCGAGGA CGCATAGAGA GACAATGACT

          260         270         280         290         300
TCCAGTCTTG GCTGGGTCCG CTGCAGTTCC TGTCTCCTGT ATTCACTAAC
AGGTCAGAAC CGACCCAGGC GACGTCAAGG ACAGAGGACA TAAGTGATTG

          310         320         330    Hind III   350
TCCCTGGTTT TTGGTACTTC TGACCGCGTT TACCAGAAGC TTAAAGACCT
AGGGACCAAA AACCATGAAG ACTGGCGCAA ATGGTCTTCG AATTTCTGGA

          350         370         380         390         400
CGAAGAAGGC ATCCTGGCTC TGATGCGTGA ACTGGAAGAC GGTACCCCAC
CCTTCTTCCG TAGGACCGAG ACTACGCACT TGACCTTCTG CCATGGGGTG

          410         420         430         440         450
GCGCAGGTCA GATCCTGAAA CAAAACTTATG ACAAATTCGA TACTAACATG
CGCGTCCAGT CTAGGACTTT GTTTGAATAC TGTTTAAGCT ATGATTGTAC

          460         470         480         490         500
CCTTCTGACG ACGGTCTGCT GAAAAACTAC GGTTTACTGT CCTGCTTCCG
GGAAGACTGC TGCCAGACGA CTTTTTGATG CCAAATGACA GGACGAAGGC

          510         520         530         540         550
CAAAGATCTG CATAAGACTG AAACCTACCT GCCTGTAATG AAATGTCGTC
GTTTCTACAC GTATTCTGAC TTTGGATGGA CGGACATTAC TTTACAGCAG

          560         570         580         590
CTTTTGGTGA AGCATCTTGC CCATTCTAAG GATCCTAATA C
CAAAACCACT TCGTAGAACG GGTAAGATTC CTAGGATTAT CAGCT
```

Sal I end

TABLE 2

## TABLE 3

```
            1                                                    10
    met     ala phe pro ala met ser leu ser gly leu phe ala
TAGAGAATG   GCT TTT CCA GCA ATG TCT CTG TCC GGT CTG TTC GCT


                            20
asn ala val leu arg ala gln his leu his gln leu ala ala asp
AAC GCG GTA CTG CGT GCT CAG CAT CTG CAC CAG TTA GCC GCG GAC


        30                                        40
thr phe lys glu phe glu arg thr tyr ile pro glu gly gln arg
ACT TTC AAA GAA TTT GAA CGT ACC TAC ATC CCA GAA GGT CAA CGC


                            50
tyr ser ile gln asn thr gln val ala phe cys phe ser glu thr
TAC TCT ATC CAG AAC ACT CAG GTT GCT TTC TGC TTT TCT GAG ACT


        60                                        70
ile pro ala pro thr gly lys asn glu ala gln gln lys ser asp
ATT CCG GCA CCA ACC GGT AAA AAC GAG GCA CAG CAG AAA TCC GAT


                            80
leu glu leu leu arg ile ser leu leu leu ile gln ser trp leu
CTG GAG CTC CTG CGT ATC TCT CTG TTA CTG ATC CAG TCT TGG CTG


        90                                       100
gly pro leu gln phe leu ser arg val phe thr asn ser leu val
GGT CCG CTG CAG TTC CTG TCT CGT GTA TTC ACT AAC TCC CTG GTT


                           110
phe gly thr ser asp arg val tyr glu lys leu lys asp leu glu
TTT GGT ACT TCT GAC CGC GTT TAC GAG AAG CTT AAA GAC CTG GAA


        120                                      130
glu gly ile leu ala leu met arg glu leu glu asp gly thr pro
GAA GGC ATC CTG GCT CTG ATG CGT GAA CTG GAA GAC GGT ACC CCA


                           140
arg ala gly gln ile leu lys gln thr tyr asp lys phe asp thr
CGC GCA GGT CAG ATC CTG AAA CAA ACT TAT GAC AAA TTC GAT ACT


        150                                      160
asn met arg ser asp asp ala leu leu lys asn tyr gly leu leu
AAC ATG CGT TCT GAC GAC GCT CTG CTG AAA AAC TAC GGT TTA CTG


                           170
ser cys phe arg lys asp leu his lys thr glu thr tyr leu arg
TCC TGC TTC CGC AAA GAT CTG CAT AAG ACT GAA ACC TAC CTG CGT


        180                                  190 191
val met lys cys arg arg phe gly glu ala ser cys ala phe OC
GTA ATG AAA TGT CGT CGT TTT GGT GAA GCA TCT TGC GCA TTC TAA

GGATCCTAATAG
```

## Example 2

Construction of $bGH_{1\text{-}32,\ 40\text{-}191}$:

A $bGH_{1\text{-}32,\ 40\text{-}191}$ analog was constructed by ligating the large fragment (SST II to Bam HI) of pCFM414bGH to the small fragment (Hinc II to Bam HI) from pbGH Syn 2-2 and the phosphorylated synthetic linker,

```
5'GGACACTTTCAAAGAATTTGGTC3'

3'CGCCTGTGAAAGTTTCTTAAACCAG5'
```

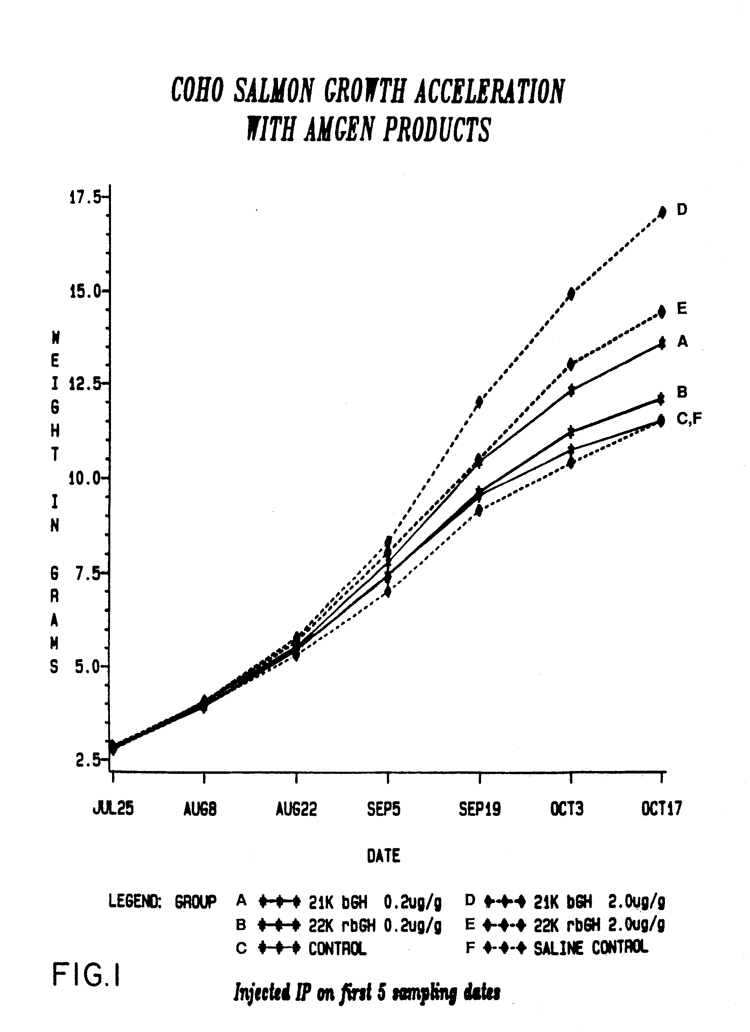

in a three-way ligation, to yield the plasmid pCFM414bGH (Figure 1).

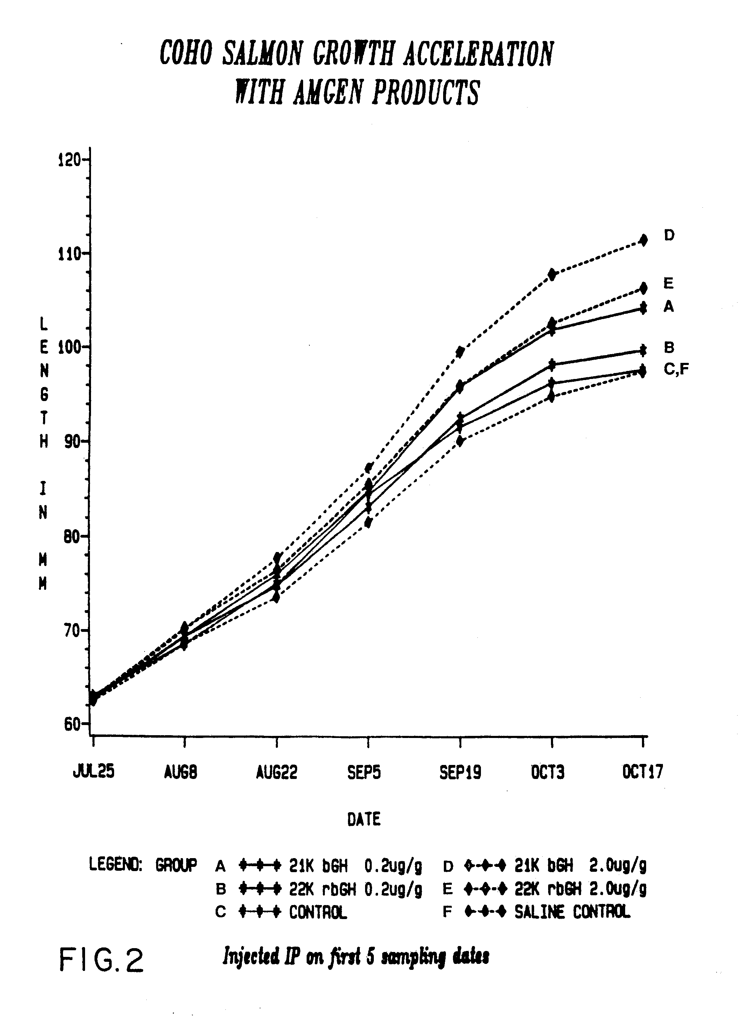

To construct the pCFM756nsbGH21K plasmid a four-way ligation was required (Figure 2). Component 1 was a ds DNA oligonucleotide with an AatII restriction site at one end and an NdeI site at the other as follows:

```
5'      CAGATCCATAAATTATCTCTGGCGGTGTTGACATAAATAC-

3'  TGCAGTCTAGGTATTTAATAGAGACCGCCACAACTGTATTTATG-
```

```
-CACTGGCGGTGATAATGAGCACATCGATTTGATTCTAGAAGGAGGAATAACA      5'

-GTGACCGCCACTATTACTCGTGTAGCTAAACTAAGATCTTCCTCCTTATTGTAT  3'
```

Component 2 was isolated from a mpII/bGH/NdeI plasmid as a NdeI to SstII ds DNA fragment containing the 5' terminal end of the bGH gene. To construct the mpII/bGH/NdeI plasmid, a site specific mutagenesis was carried out on a mpII/bGH plasmid to create an NdeI site (CATATG) where the ATG in the NdeI site would be the methionine amino acid #1 of the bGH gene.

Component 3 was isolated from a pCFM414bGH21K plasmid as a SstII to BamHI ds DNA fragment containing the 3' end of the bGH21K gene.

Component 4 was a pCFM736ns plasmid cut with AatII and BamHI. The pCFM736ns plasmid is a derivative of the pCFM736 plasmid (described below) prepared by inserting the following sequence at the unique BamHI site:

```
5'   GATCCGCGGATAAATAAGTAAC       3'

3'        GCGCCTATTTATTCATTGCTAG   5'
```

The plasmid pCFM736 is prepared as a derivative of pCFM536 (ATCC# 39934) constructed to incorporate a Kanamycin resistance marker, and a synthetic P1 promoter. The B-lactamase gene is first deleted by digestion of pCFM536 with SstI and XbaI. This serves to delete not only the marker gene but also the entire "par" or stability sequence, the P1 promoter, and part of the cluster of restriction sites. The Kanamycin gene sequence may be obtained as a SmaI to HindIII fragment from the Tn5 plasmid of Beck et al., Gene 19, pp. 327-336 (1982) or Auerswald et al., Cold Spring Harbor Symp. Quant. Biol., 45, pp. 107-113 (1981). To prepare the fragment for insertion into the new vector, a SstI linker is added to the SmaI site and an NdeI linker added to the HindIII site. The "par" locus sequence may be obtained as a HincII to AvaI digestion fragment of PSC101 (ATCC#37032). To prepare the "par" fragment for insertion into the new vector, the HincII is first treated with a SalI linker and then an AatII linker. The AvaI site is treated with a BamHI linker and then an NdeI linker. A DNA sequence containing a synthetic P1 promoter obtained by chemical synthesis of a ds DNA oligonucleotide with sticky ends for insertion between an AatII restriction site and an XbaI restriction site was added as follows:

```
5'      CAGATCCATAAATTATCTCTGGCGGTGTTGACATAAATAC-

3'  TGCAGTCTAGGTATTTAATAGAGACCGCCACAACTGTATTTATG-
```

```
-CACTGGCGGTGATAATGAGGACATCGATT       3'
                                          .
-GTGACCGCCACTATTACTCGTGTAGCTAAGATC  5'
```

After ligation the plasmid construction (now called pCFM756nsbGH21K) was transformed into E. coli cells of strain FM6 (source/deposit). FM6 is a derivative of AM7 (#CG608159) that has been rendered phage resistant to several unknown bacteriophages and contains the gene encoding tetracycline resistance and the lambda bacteriophage repressor genes, CI857 and cro, integrated into the chromosome.

The bGH$_{1-32, 40-191}$ analog was isolated from a strain of Escherichia coli, FM6, carrying a ts runaway plasmid into which the appropriate gene sequence, along with a trp promoter system, had been inserted. Biologically active bGH$_{1-32, 40-191}$ analog was recovered after breakage of harvested cells with a Manton-Gaulin press. The growth hormone was present, in insoluble form, in a pellet fraction obtained by centrifugation of the cell lysate. The broken cell pellet fraction was extracted using deoxycholate, EDTA and lysozyme. The bGH$_{1-32, 40-191}$ analog in the extracted pellet was solubilized using 6M guanidine-HCl in Tris buffer at pH 8.5. It was further purified by gel filtration using a Sephacryl S-200 column equilbrated in 6M guanidine-HCl, 50 mM Tris-HCl H 8.5. The bGH$_{1-32, 40-191}$ analog eluting in an included peak from the column was dialyzed against a buffer of 0.2 percent (w/v) lactose, 0.2 percent (w/v) mannitol, 0.25 percent (w/v) sodium bicarbonate, pH 8.5. Precipitated material that appeared during the dialysis was removed by centrifugation and the preparation was concentrated by ultrafiltration and lyophilized.

The resulting bGH$_{1-32, 40-191}$ preparation was greater than 90% pure, as judged by densitometric scanning of sodium dodecyl sulfate (SDS) polyacrylamide gels stained with Coomassie blue R250. Similar to natural growth hormones, bGH$_{1-32, 40-191}$ analog was either monomeric or dimeric in structure as determined by gel filtration carried out in the lactose-mannitol-sodium bicarbonate buffer. Based upon the results of gel filtration and SDS polyacrylamide gel electrophoresis (PAGE) under non-reducing conditions, the bGH$_{1-32, 40-191}$ analog was essentially devoid of high molecular weight aggregated forms (i.e. these forms represented less than 2% of the rbGH). The bGH$_{1-32, 40-191}$ analog elutes somewhat earlier than nbGH from reversed phase HPLC in migration on isoelectric focusing gels, and in levels of free thiol detected by the method of Ellman, Arch.Biochem. & Biophys., 82, 70-77 (1959). Free thiol levels were less than 0.1 mole per mole of hormone monomer, as is expected for a hormone with a native configuration, since all known natural growth hormones have two intra-chain disulfide bonds and no free cysteine residues. The amino acid sequence of rbGH$_{1-191}$,(22K rbGH) is given in Table 4.

TABLE 4

```
bGH                                                                                                    ATG ATG
                                                                                                       met met

                                  -20                                           -10                      -1   1
bGH     ACC GCA GGC CCC CGG ACC TCC CTG CTC CTG GCT TTC GCC CTG CTC TGC CTG CCC TGG ACT CAG GTG GTG GGC GCC
        ala ala gly pro arg thr ser leu leu leu ala phe ala leu leu cys leu pro trp thr gln val val gly ala

                                              10                                        20
bGH     TTC CCA GCC ATG TCC TTG TCC GGC CTG TTT GCC AAC GCT GTG CTC CGG GCT CAG CAC CTG CAT CAG CTG GCT GCT
        phe pro ala met ser leu ser gly leu phe ala asn ala val leu arg ala gln his leu his gln leu ala ala

                          30                                      40                                  50
bGH     GAC ACC TTC AAA GAG TTT GAG CGC ACC TAC ATC CCG GAG GGA CAG AGA TAC TCC ATC CAG AAC ACC CAG GTT GCC
        asp thr phe lys glu phe glu arg thr tyr ile pro glu gly gln arg tyr ser ile gln asn thr gln val ala

                                          60                                      70
bGH     TTC TGC TTC TCT GAA ACC ATC CCG GCC CCC ACG GGC AAG AAT GAG GCC CAG CAG AAA TCA GAC TTG GAG CTG CTT
        phe cys phe ser glu thr ile pro ala pro thr gly lys asn glu ala gln gln lys ser asp leu glu leu leu

                          80                                      90                                  100
bGH     CGC ATC TCA CTG CTC CTC ATC CAG TCG TGG CTC GGG CCC CTG CAG TTC CTC AGC AGA GTC TTC ACC AAC AGC TTG
        arg ile ser leu elu leu ile gln ser trp leu gly pro leu gln phe leu ser arg val phe thr asn ser leu

                                          110                                     120
bGH     GTG TTT GGC ACC TCG GAC CGT GTC TAT GAG AAG CTG AAG GAC CTG GAG GAA GGC ATC CTG GCC CTG ATG CGG GAG
        val phe gly thr ser asp arg val tyr glu lys leu lys asp leu glu glu gly ile leu ala leu met arg alu

                          130                                     140                                 150
bGH     CTG GAA GAT GGC ACC CCC CGG GCT GGG CAG ATC CTC AAG CAG ACC TAT GAC AAA TTT GAC ACA AAC ATG CGC AGT
        leu glu asp gly thr pro arg ala gly gln ile leu lys gln thr tyr asp lys phe asp thr asn met arg ser

                                          160                                     170
bGH     GAC GAC GCG CTG CTC AAG AAC TAC GGT CTG CTC TCC TGC TTC CGG AAG GAC CTG CAT AAG ACG GAG ACG TAC CTG
        asp asp ala leu leu lys asn tyr gly leu leu ser cys phe arg lys asp leu his lys thr glu thr tyr leu

                          180                                     191
bGH     AGG GTC ATG AAG TGC CGC CGC TTC GGG GAG GCC AGC TGC GCC TTC
        arg val met lys cys arg arg phe gly glu ala ser cys ala phe
```

0 282 318

Example 3

The following analogs may be constructed using the above procedure but substituting different phosphorylated synthetic linkers:

$\underline{bGH}_{1-35, \ 39-191}$

```
              5'GGACACTTTCAAAGAATTTGAACGTACCGAAGGTC3'
linker:       3'CGCCTGTGAAAGTTTCTTAAACTTGCATGGCTTCCAG5'
```

$\underline{bGH}_{1-37, \ 39-191}$

```
              5'GGACACTTTCAAAGAATTTGAACGTACCTACATCGAAGGTC3'
linker:3'CGCCTGTGAAAGTTTCTTAAACTTGCATGGATGTAGCTTCCAG5'
```

$\underline{bGH}_{1-32, \ 35-38, \ 40-191}$

```
              5'GGACACTTTCAAAGAATTTACCTACATCCCAGGTC3'
linker:       3'CGCCTGTGAAAGTTTCTTAAATGGATGTAGGGTCCAG5'
```

$\underline{bGH}_{1-33, \ 35-191}$

```
              5'GGACACTTTCAAAGAATTTGAAACGTACATCCCAGAAGGTC3'
linker:3'CGCCTGTGAAAGTTTCTTAAACTTTGCATGTAGGGTCTTCCAG5'
```

Example 4

The bGH$_{1-32, \ 40-191}$ analog was evaluated in a radioimmunoassay for ruminant growth hormone according to the procedure of Hart, et al., Horm. Metab. Res., 7, 35-40, (1975) with modifications described by Tindal, et al., Horm. Metab. Res., 14, 425-429, (1982).

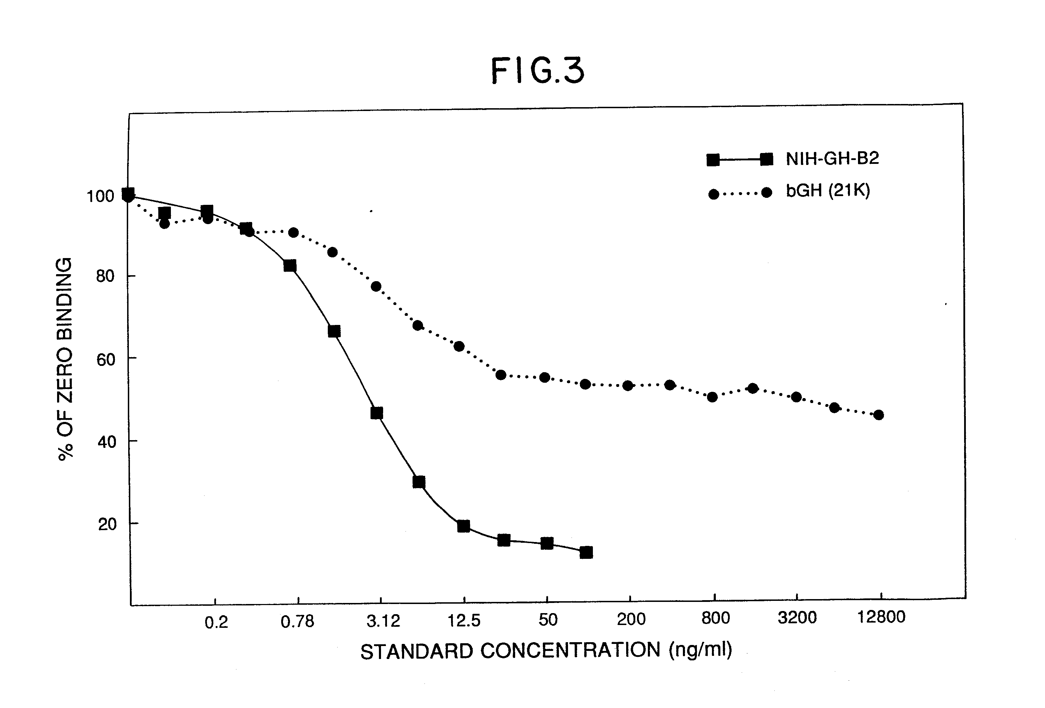

Non-parallel cross-reactions and incomplete competition were noted for recombinant bGH$_{1-32, \ 40-191}$ analog in the radioimmunoassay for bovine growth hormone as indicated by the character, i.e., differences in slope and zero percent binding of the lines in Fig. 3.

The data shown is Figure 3 can be interpreted as follows: 1) 21KbGH shares some common antigenic determinants with native bGH; 2) both molecules share related antigenic sites (subtle structural differences yielding different affinities); and 3) some structural components of native bGH are not present on 21KbGH.

Example 5

Growth promoting activity of the bGH analog preparations of the present invention was measured by the dwarf mouse assay [Wallis et al., J. Endocrinol., 56, 235-243 (1973)]. Recombinant bGH$_{1-191}$ is growth promoting and had an activity of 1.4U/mg in this assay [see Hart, et al., Biochem. J., 224, 93-100 (1984).

The results for dwarf mouse assays of the bGH$_{1-32, \ 40-191}$ analog and of naturally occurring bGH control were as follows:

## TABLE 5

| | Treatment ( μ g/d) | Wt. Gain Over 26 Days (g) |
|---|---|---|
| Control | | |
| Standard bovine growth hormone (NIH-GH-B15; U/mg) | 10 | 1.9 |
| | 40 | 2.9 |
| | 160 | 3.9 |
| bGH$_{1-32, 40-191}$ | 20 | 3.4 |
| | 80 | 4.8 |

### Example 6

Recombinant bovine growth hormone preparations were compared in a hypophysectomized rat bioassay for weight gain.

The animals used in the bioassay were female Sprague-Dawley rats (Charles River, Portage, WA) weighing 100-110 grams at hypophysectomy. The rats were housed at 4-5 per hanging wire cage. The animals were not provided with any supplements from arrival to the beginning of study. Baseline body weights were recroded over a 7-11 day period; then rats were grouped randomly (9-10/group). One subcutaneous injection of 0.1 ml/rat was administered daily for 10 consecutive days.

The day after the last injection, the rats are weighted a final time and the average weight calculated for each dose group. The average weight gain for the buffer control group is subtracted from each treatment group. The results of the first experiment (10 animals/group) are depicted in Table 6. This experiment included two independent samples of rbGH22K (Samples A, B) and one sample of rbGH21K (21K Sample C). The work was repeated in a second experimental protocol (9 rats/group) in which two samples of rbGH21K (21K Sample C, 21K Sample D), one sample of rbGH Sample A and a pituitary bGH preparation were compared (Table 6). In both experimental protocols, the recombinant GH preparations were tested at three doses (30, 100, 300 μ g/Kg).

In the second experiment the potencies of the various GH preparations were calculated using bGH 22K Sample A as the "standard" with a relative potency of 1U/mg. The individual body weight changes for each rat were entered into this regression equation [dose relationship between log (dose) and weight gain] and averaged at each dose per lot. The curves for rbGH 21K sample D were not parallel with that of 22K Sample A; so while these were clearly more potent than 22K Sample A, there was a lot of variance across the three doses tested. The higher doses of 22K Sample C and 22K Sample D produced significantly more growth than 21K Sample A; so the estimated "equivalents" arose by extrapolating far beyond the end of the 21K Sample A curve to 160-200 μg/day doses at which point the curve may not be linear with the lower doses. The dose of pituitary GH tested falls within the range of the 21K Sample A curve. The unit equivalents to rbGH 21K Sample A are presented in Table 5 for the second experiment. The data demonstrate that rbGH21K is 2-4 times more potent than "unmodified" rbGH22K or pituitary bGH in a hypophysectomized rat weight gain model.

### Example 7

Injection of bGH$_{1-32, 40-191}$ analog into juvenile coho salmon (Oncorhynchus kisutch) results in significant dose dependent increases in growth rate. Gill et al., Bio/Technology, 3, 643-646 (1985). The following experiment was performed to compare the bGH$_{1-32, 40-191}$ analog with bGH$_{1-191}$.

Small Coho salmon (about 3g each), obtained from the Capilano Salmon Hatchery, British Columbia, Canada were randomly distributed, in groups of 120, 60 per 200 litre fiberglass tank. The fibreglass tanks were supplied with aerated, running well water. The salmon were maintained indoors under a simulated natural photoperiod. Water temperature was 10-11°C. during the experiment.

## TABLE 6

### HYPOX RAT (WT. GAIN)

| Treatment Groups | Dose ($\mu$G/Rat/Day | Wt. Gain Exp #1 (G) | Wt. Gain Exp #2 (G) |
|---|---|---|---|
| Control | -- | 2.2 | 3.8+/-1.0 |
| Pituitary bGH | 10 | NT[1] | 10.5+/-1.3 |
| rbGH 21K (Sample B) | 3 | 6.5 | NT |
| | 10 | 9.8 | NT |
| | 30 | 15.1 | NT |
| rbGH 21K (Sample A) | 3 | 7.4 | 4.7+/-0.6 |
| | 10 | 11.1 | 9.1+/-1.6 |
| | 30 | 17.3 | 14.6+/-1.2 |
| $bGH_{1-32, 40-191}$ | 3 | 12.3 | 9.2+/-0.8 |
| rbGH 21K (Sample C) | 10 | 19.1 | 16.0+/-1.2 |
| | 30 | 25.1 | 22.4+/-1.8 |
| rbGH 21K (Sample D) | 3 | NT | 7.4+/-1.1 |
| | 10 | NT | 13.5+/-0.8 |
| | 30 | NT | 21.8+/-1.2 |

NT[1] - Not Tested

## TABLE 7

### Growth Hormone

### Unit Equivalents to Lot r—bGH 21K (Sample A)

| Sample | U/mg | # Doses |
|---|---|---|
| rbGH 21K (Sample A) | 1.03 ± 0.02 | 3 |
| rbGH 21K (Sample C) | 4.62 ± 0.99 | 3 |
| rbGH 21K (Sample D) | 3.34 ± 1.15 | 3 |
| Pituitary bGH | 1.23 | 1 |

Fish were fed a dry diet (West Van 33, moisture content between 8 and 9%) to satiation twice daily. The size of food particles was adjusted to the mean weight of the fish to obtain maximal growth rates.

Fish were acclimated to these conditions for 14 days before beginning hormone administration. Once every two weeks the fish were anaesthetized in 2-phenoxyethanol (1 in 10,000), weighed to the nearest 0.01 g, measured to the nearest 0.1 cm, and injected intraperitoneally with $bGH_{1-191}$ or $bGH_{1-32, 40-191}$ analog in a buffer of 0.65 percent (w/v) NaCl, 1 percent (w/v) bovine serum albumin such that 50 µl contained a dose equivalent to either 0.2 or 2 µg/g body weight. Each week, the dosage of hormone was recalculated to allow for growth.

Members of a first control group were injected with the buffer while members of a second control group were not injected. Results are shown as mean values. Results were analyzed by unbalanced one-way Analysis of Variance (BMDP statistical package), followed by Bonferroni's multiple range test to determine levels of significance between treatments.

As shown in Figs. 1 and 2, fish treated with a $bGH_{1-32, 40-191}$ analog exhibited a growth advantage as compared to the fish treated with $bGH_{1-191}$ treated fish. This difference is statistically valid $P < .0001$.

Example 8

Six ewes were treated in pairs, with each of three preparations [nbGH, $rbGH_{1-191}$ and $rbGH_{1-32, 40-191}$ analog] being used in a different pair in each treatment period. Each treatment period consisted of a single daily subcutaneous injection of test material (0.2 mg/kg liveweight per day; 1 mg/ml lactose, mannitol, bicarbonate buffer, pH 8.5-9.0) on each of 8 days separated by 10-day periods when ewes received similar daily injections of buffer only.

Treatments began between days 32-40 of lactation when the yields of all ewes were beginning to decline, and the yields continued to decline during each successive control period. Ewes were fed a restricted amount of concentrate and chopped hay twice each day so that food intake remained constant throughout the experiment. Only one ewe (No. 553) failed to consume its concentrate allowance on all occasions.

The response in daily milk yield in six Dorset ewes following daily injections of various preparations of bovine growth hormone for 8-day treatment periods is presented in Table 9.

In Table 9, the response is the mean milk yield in the last four days of bGH injection minus the mean yield in the four days immediately preceding the commencement of treatment as expressed in terms of weight (g) of additional milk, or in terms of a percentage increase.

In general, the yield responses were higher than anticipated, based on the results of previous work and on preliminary dose response investigations in two spare ewes at the beginning of lactation. A relatively high dose (0.2 mg/kg liveweight) was chosen and in most cases significant increases in yield were achieved. Statistical analysis is difficult because: (i) as milk yield declines during lactation, the response is being measured relative to a changing control baseline; and (ii) it is suspected that the responsiveness of the animal to exogenous growth hormone increases as lactation advances. It is also obvious that in some cases eight days of injections was not sufficient to reach a plateau in milk yield and, where a plateau was established, there is no way of knowing whether the yields

## TABLE 9

| Ewe Number | Pit. bGH Yield (g) | Pit. bGH Increase (%) | rbGH$_{1-191}$ Yield (g) | rbGH$_{1-191}$ Increase (%) | rbGH$_{1-32, 40-191}$ Yield (g) | rbGH$_{1-32, 40-191}$ Increase (%) |
|---|---|---|---|---|---|---|
| 542 | 705 | 40 | 560 | 26 | 680 | 27 |
| 543 | 155 | 9 | 400 | 22 | 590 | 42 |
| 544 | 460 | 34 | 400 | 27 | 400 | 23 |
| 545 | 110 | 5 | 560 | 38 | 355 | 20 |
| 552 | 760 | 41 | 805 | 48 | 945 | 54 |
| 553 | 255 | 15 | 415 | 21 | 450 | 26 |
| Total | 2445 | | 3140 | | 3420 | |

15

might have increased further had treatment been continued. A preliminary summary, based upon a simple comparison between mean 4-day yield before and at the end of each treatment period, indicates a marginal advantage in response to $rbGH_{1-32, 40-191}$ and $rbGH_{1-191}$ over the pituitary bGH preparation used, due mainly to a greater consistency of response between the individual animals when given the recombinant material (see Table 5).

The features disclosed in the foregoing description, in the following claims and/or in the accompanying drawings may, both separately and in any combination thereof, be material for realising the invention in diverse forms thereof.

**Claims**

1. A bovine growth hormone analog comprising the amino acid sequence
$Z$-$bGH_{1-32}$-$(X)_n$-$bGH_{40-191}$
wherein
n is 0 or 1;
Z is hydrogen or methionine; and
X is a peptide of an amino acid residue comprising

-GLU-ARG-THR-TYR-ILE-PRO-GLU-
wherein one or more amino acids are deleted; and allelic versions thereof.

2. A bovine growth hormone analog according to Claim 1 wherein n is 0.

3. A bovine growth hormone analog according to Claim 1 wherein n is 1 and X is
-GLU-ARG-THR-GLU-;
-THR-TYR-ILE-PRO-;
-GLU-ARG-THR-TYR-ILE-GLU-;
-GLU-THR-TYR-ILE-PRO-GLU-.

4. A DNA sequence comprising a sequence encoding a bovine growth hormone represented by the formula
$Z$-$bGH_{1-32}$-$(X)_{\overline{n}}$-$bGH_{40-191}$
wherein
n is 0 or 1;
Z is hydrogen or methionime; and
X is a peptide of an amine acid residue comprising

-GLU-ARG-THR-TYR-ILE-PRO-GLU-
wherein one or more amino acids are deleted; and allelic versions thereof.

5. An expression vehicle capable, in a transfected all culture of expressing a DNA sequence according to Claim 4.

6. A cell culture transfected with an expression vehicle according to Claim 5.

7. A microorganism according to Claim 6 obtained by transfecting an E. coli strain.

8. A composition comprising a bovine growth hormone analog represented by the amino acid sequence
$z$-$bGH_{1-32}$-$(X)_{\overline{n}}$-$bGH_{40-191}$
wherein
n is 0 or 1;
Z is hydrogen or methionine; and
X is a peptide of an amino acid residue comprising

-GLU-ARG-THR-TYR-ILE-PRO-GLU-
wherein one or more of the amino acids are deleted; and allelic versions thereof and essentially free of other proteins of bovine origin.

9. A method for promoting growth in an animal comprising administering to an animal an effective dose of a bovine growth hormone analog of Claim 1.

10. A method of promoting milk production in an animal comprising administering to the animal an effective dose of a bovine growth hormone analog of Claim 1.

0282318

# COHO SALMON GROWTH ACCELERATION
# WITH AMGEN PRODUCTS

LEGEND: GROUP A ◆—◆—◆ 21K bGH 0.2ug/g    D ◆—◆—◆ 21K bGH 2.0ug/g
        B ◆—◆—◆ 22K rbGH 0.2ug/g    E ◆—◆—◆ 22K rbGH 2.0ug/g
        C ◆—◆—◆ CONTROL             F ◆—◆—◆ SALINE CONTROL

FIG.1

*Injected IP on first 5 sampling dates*

0282318

# COHO SALMON GROWTH ACCELERATION
# WITH AMGEN PRODUCTS

DATE

LEGEND: GROUP  A ✦–✦–✦ 21K bGH  0.2ug/g   D ✧–✦–✦ 21K bGH  2.0ug/g
                B ✦–✦–✦ 22K rbGH 0.2ug/g   E ✦–✧–✦ 22K rbGH 2.0ug/g
                C ✦–✦–✦ CONTROL            F ✦–✦–✦ SALINE CONTROL

FIG.2          *Injected IP on first 5 sampling dates*

# FIG.3

FIG.4

M13 mp11 containing bGH gene

<u>Site directed mutagenesis</u> to create NdeI site (CATATG) where the ATG in the site would correspond to the methionine amino acid #1 of the bGH gene

M13 mp11/bGH/NdeI

Component 1:
AatII-NdeI DNA
Oligonucleotide
Fragment

Component 2:
NdeI-SstII DNA
Fragment from M13 mp11/bGH/NdeI

AatII

NdeI

SstII

Component 3:
SstII-BamHI DNA
Fragment from
pCFM414 bGH 21K

BamHI

Component 4:
AatII-BamHI DNA
Fragment from
pCFM736ns

Ligation of fragments

Synthetic
promoter

bGH 21K gene

par

Transcription
Stop

APHII
Gene

Plasmid
Replication
Genes

ori

pcFM756ns bGH 21K

FIG.5